**Europäisches Patentamt**

**European Patent Office**

**Office européen des brevets**

(19)

(11) Numéro de publication : **0 346 245 B1**

(12)

# FASCICULE DE BREVET EUROPEEN

(45) Date de publication du fascicule du brevet :
**07.10.92 Bulletin 92/41**

(51) Int. Cl.$^5$ : **A61F 2/16**

(21) Numéro de dépôt : **89420161.5**

(22) Date de dépôt : **28.04.89**

(54) **Implant intra-oculaire de chambre antérieure.**

(30) Priorité : **11.05.88 FR 8807042**

(43) Date de publication de la demande :
**13.12.89 Bulletin 89/50**

(45) Mention de la délivrance du brevet :
**07.10.92 Bulletin 92/41**

(84) Etats contractants désignés :
**AT BE CH DE ES FR GB GR IT LI LU NL SE**

(56) Documents cités :
**EP-A- 0 195 881
EP-A- 0 215 468
FR-A- 2 530 457
FR-A- 2 603 186
US-A- 2 834 023
US-A- 4 504 981**

(73) Titulaire : **LABORATOIRES DOMILENS
321 avenue Jean Jaurès
F-69007 Lyon (FR)**

(72) Inventeur : **Baikoff, Georges Bâtiment D
Résidence Les Alpilles Corniche J Kennedy
F-13008 Marseille (FR)**

(74) Mandataire : **Guerre, Dominique et al
CABINET GERMAIN et MAUREAU Le
Britannia Tour C 20 Bd E. Deruelle
F-69392 Lyon Cédex 03 (FR)**

Jouve, 18, rue Saint-Denis, 75001 PARIS

## Description

La présente invention a pour objet un implant intra-oculaire de chambre antérieure, destiné à corriger les myopies optiques et ce par intervention chirurgicale de l'oeil.

Selon le document FR-A-2 530 457, et plus précisément selon les figures 5 à 9 de ce dernier, on a décrit un implant comprenant de manière distincte, en ce qui concerne la construction et les matériaux utilisés :

– une partie optique en verre comprenant une lentille divergente à bords épais, de forme essentiellement circulaire, occupant substantiellement en totalité la partie optique, et présentant une face antérieure et une face postérieure

– une partie haptique élastique, permettant un appui à l'intérieur de l'oeil, et s'étendant à distance de la partie optique ; cette partie haptique comporte un anneau périphérique élastique, par exemple en acier inoxydable, et des sangles radiales d'attache de la partie optique, par exemple en polypropylène.

Un tel implant présente les inconvénients suivants.

Les parties optique et haptique ne sont pas construites de manière monobloc, et en un seul et même matériau transparent, comme le sont par exemple les parties optique et haptique d'un implant à lentille convergente selon les figures 2 et 3 du document FR-A-2 530 457. Ceci complique la réalisation de l'implant et rend plus difficile sa mise en place dans l'oeil.

Et la lentille divergente présente des bords peu épais, ce qui veut dire que sa puissance optique négative demeure relativement faible.

Selon le document EP-A-0 215 468, on a décrit un implant de construction monobloc, comprenant une partie optique à lentille convergente, et une partie haptique avec anses opposées. Cet implant présente de ce fait une bordure circulaire ayant sensiblement la même épaisseur que celle des anses.

Un tel implant présente les inconvénients suivants.

Il est nécessaire d'affecter une partie de la surface de la partie optique au raccordement avec les anses, ce qui conduit à la fois à diminuer la surface utile de la lentille, et à apporter dans le champ optique de l'oeil des signaux parasites.

La zone de raccordement des anses à la partie optique présente une largeur diminuant progressivement jusqu'à la partie terminale desdites anses. Ceci augmente la rigidité de ces dernières, et gène la mise en place dans l'oeil de l'implant.

La présente invention a donc pour objet un implant avec lentille divergente, de construction simplifiée, et préservant tant à la fois le maximum de surface à la lentille divergente et une bonne souplesse à la partie haptique.

Conformément à la présente invention, la partie optique étant construite de manière monobloc avec la partie haptique, en un matériau transparent, d'une part, la partie haptique comprend deux anses d'appui à l'intérieur de l'oeil, s'étendant de part et d'autre de la partie optique, selon la même direction diamétrale, à partir de deux points de la partie optique respectivement et diamétralement opposés, et d'autre part, une zone de raccordement de section décroissante vers l'extrémité libre de chaque anse, située à l'extérieur de la partie optique, et reportée sur chaque anse, relie cette dernière à la partie optique, ladite zone de raccordement présentant en tout point de sa surface extérieure un profil courbe.

La zone de raccordement définie précédemment permet de passer de l'épaisseur des anses d'appui élastique, à l'épaisseur des bords épais de la partie optique ou lentille, par exemple de l'ordre de deux à dix fois supérieure à la première. Avec un tel rapport d'épaisseurs, il devient possible de faire varier la puissance de la lentille divergente, par exemple entre - 10 et - 70 dioptries, tout en conservant l'élasticité des anses. d'appui de l'implant.

Afin de limiter au maximum la longueur de la zone de raccordement, la longueur de cette dernière n'excède pas 10 % de la longueur totale de chaque anse d'appui élastique. Ceci permet en particulier de préserver pour l'essentiel la conformation élastique de chaque anse d'appui, sans apporter de rigidité excessive à cette dernière.

Selon un mode préféré d'éxécution de l'invention, la zone de raccordement présente un bossage disposé du côté de la face antérieure de la lentille, au niveau du point de départ de chaque anse d'appui élastique. Un tel bossage avec l'inclinaison correspondante des anses d'appui permet dès la naissance de ces dernières, d'écarter la partie haptique de la partie optique, et donc de limiter au maximum toute influence optique parasite de ladite partie haptique.

La présente invention est maintenant décrite par référence aux dessins annéxés, dans lesquels :

– la figure 1, comme dit plus haut, représente de manière schématique un oeil en coupe dans lequel a été implanté un implant conforme à l'invention ou selon l'art antérieur

– la figure 2 représente une vue en perspective d'un premier implant selon l'invention

– la figure 3 est une vue de face du premier implant selon l'invention

– la figure 4 est une vue de profil du premier implant selon l'invention

– la figure 5 est une vue en coupe transversale selon la ligne V-V de la figure 3 du premier implant selon l'invention

– la figure 6 est une vue de face d'un deuxième implant selon l'invention

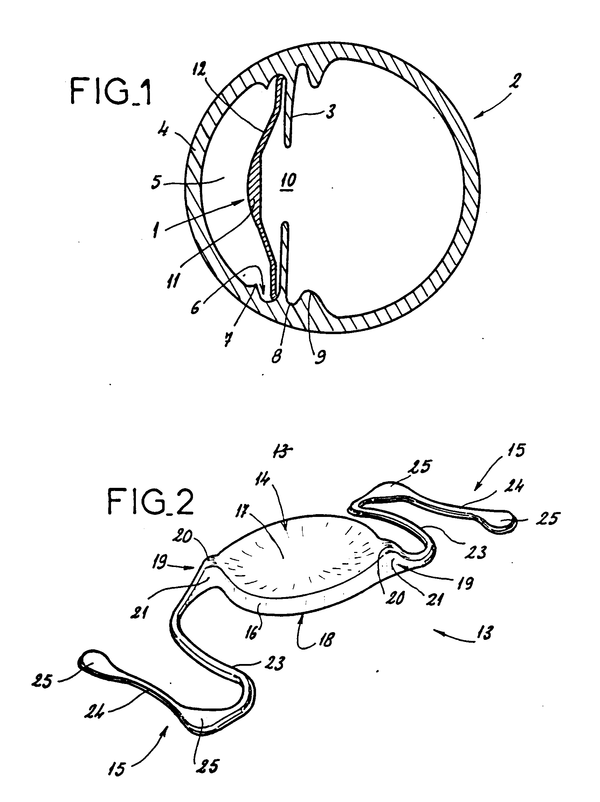

L'implant intra-oculaire représenté aux figures 2 à 5 est designé par la référence générale (13). Cet implant est obtenu par tout procédé approprié de manière monobloc, c'est à dire sans discontinuité de matière ou de composants, d'une extrémité à une autre. Il est réalisé en tout matériau transparent approprié tel que le polyméthacrylate de méthyle. Il est constitué par une partie optique (14) et par une partie haptique comportant deux anses élastiques d'appui (15).

Dans cet exemple, la partie optique (14) s'identifie pour l'essentiel à une lentille divergente biconcave à bord épais (16), de forme essentiellement circulaire, présentant une face antérieure (17) et une face postérieure (18) concaves.

Les anses élastiques d'appui s'étendent de part et d'autre de la partie optique (14), selon la même direction diamétrale (50), à partir de deux points ou zones de la partie optique, respectivement et diamétralement opposés.

Deux zones de raccordement (19), disposées à l'extérieur de la partie optique, et situées sur les deux anses d'appui élastique (15) respectivement, relient ces dernières à la partie optique, et donc assurent la jonction entre la partie optique et la partie haptique de l'implant. Chaque zone de raccordement (19) présente une section décroissante vers l'extrémité libre (25) de chaque anse, passant de l'épaisseur (e) du bord épais (16) de la partie optique (14), à l'épaisseur nominale de l'anse (15), par exemple égale à (e/4), de section transversale circulaire. La longueur de chaque zone de raccordement est aussi limitée que possible, et n'excède pas en général 10 % de la longueur totale de chaque anse, depuis son point de départ sur la partie optique (14) jusqu'à son extrémité libre (25). Comme ne le montrent pas complètement les figures, chaque zone de raccordement (19) présente en tout point de sa surface extérieure un profil courbe.

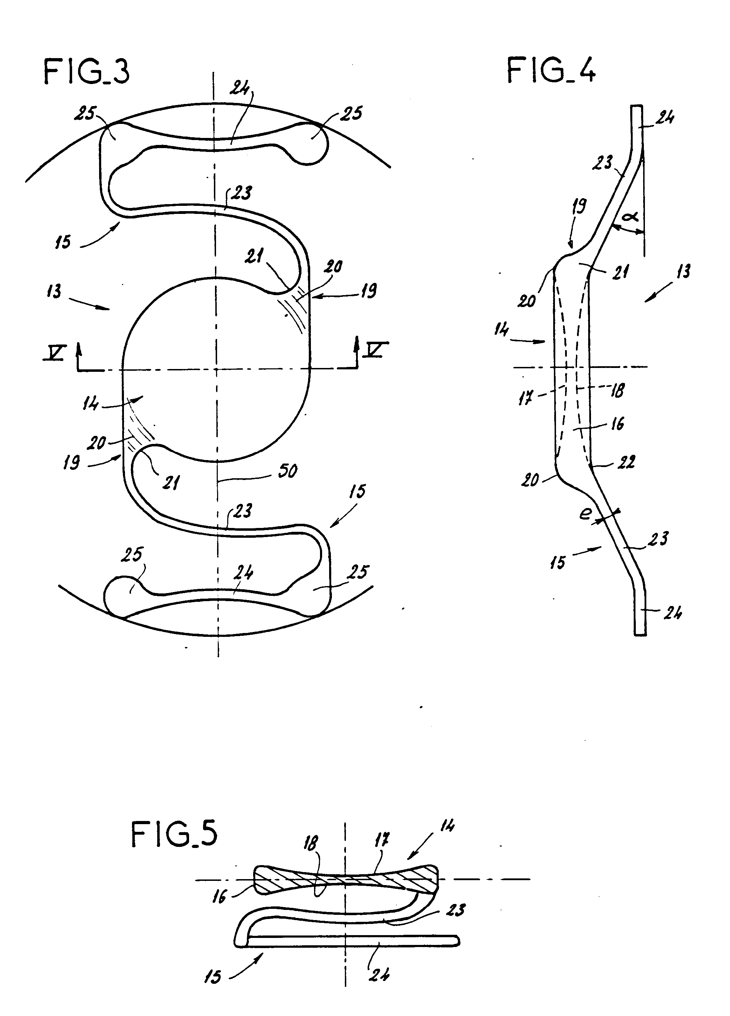

Chaque zone de raccordement (19) présente un bossage de contour arrondi, permettant de passer, sur une faible longueur, du bord épais (16) de la partie optique (14) à l'anse (15). Comme montré par la figure 4, ce bossage correspond à un arrondi.

Ainsi qu'il apparaît sur les figures 2 et3, les flancs du bossage (20) sont constitués par des prolongements du bord épais (16) en forme de parties courbées (21) de direction centrifuge par rapport à la lentille biconcave (14). Le bossage (20) présente une face intérieure (22) qui est tangente à la face postérieure (18) concave de la partie optique (14).

Chaque anse (15) comporte de manière continue, et successivement, de son point de départ sur la partie optique (14) vers son extrémité libre (25), la zone de raccordement (19), une première partie (23) courbée en arc de cercle, convexe vers l'extérieur de la lentille (14), sensiblement orthogonale à la direction diamétrale (50) d'extension de chaque anse (15), et une deuxième partie (24), courbée et convexe vers l'intérieur de la lentille (14), s'étendant en direction opposée à la première partie courbe (23), mais sensiblement parallèlement à cette dernière.

Chaque deuxième partie (24) présente à ses deux extrémités respectivement deux régions (25) de contour arrondi, destinées chacune a constituer un point d'appui de l'implant (13) dans la bande ciliaire (6) de la chambre antérieure (5) de l'oeil.

Ainsi qu'il ressort de la figure 4, les premières parties (23) courbées des anses d'appui (15) sont disposées approximativement dans un plan faisant un angle aigu $\alpha$ de l'ordre de 25° avec un plan contenant les deuxièmes parties courbées (24) des anses d'appui (15), et parallèle au plan de la partie optique (14).

L'implant intra-oculaire (13), selon ce premier mode d'exécution, présente des dimensions telles que le diamètre du cercle ayant pour centre celui de la partie optique (14) et passant par les points les plus éloignés du centre des régions (25) constituant les points d'appui de l'implant (13), autrement dit le diamètre d'enveloppe est égal 13 000 micromètres ∓ 250, le diamètre de la partie optique (14) est égal à 4 500 micromètres ∓ 130, l'épaisseur "e" des anses (15) à 200 ∓ 20 micromètres et la longueur des deuxièmes parties courbées (24) des anses (15) à 5 500 micromètres ∓ 165. Un tel implant intra-oculaire (13) est réalisé par usinage puis polissage du matériau de constitution.

Bien entendu, la lentille divergente à bord épais biconcave constituant la partie optique (14) peut également être plan-concave. Elle permet de corriger de fortes myopies et peut présenter des puissances variant de - 10 à - 60 dioptries selon les rayons de courbures des faces antérieure (17) et postérieure (18) de la partie optique (14).

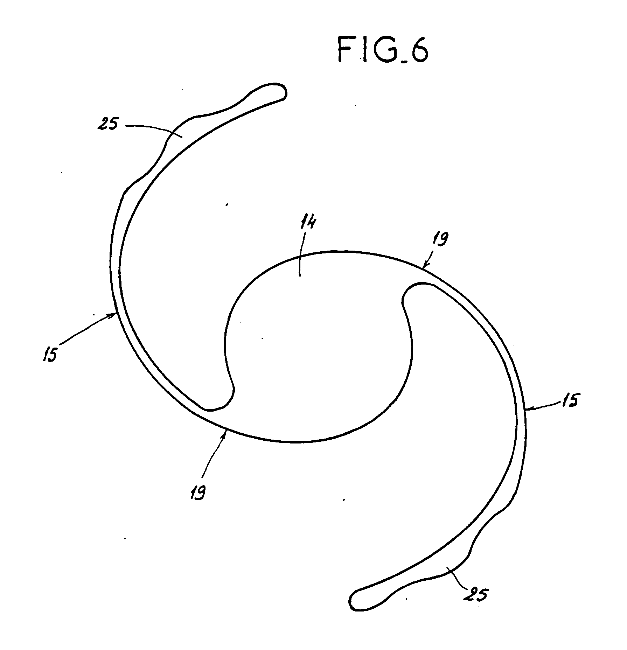

La zone de raccordement (19) peut relier la partie optique (14) à diverses anses d'appui (15) élastiques de formes différentes de celle décrite ci-dessus. Selon la figure 6, sur laquelle les références numériques communes avec celles des figures 1 à 5 désignent des éléments ayant les mêmes fonctions que celles décrites précédemment, les deux anses (15) comportent chacune une seule partie convexe tournée vers la partie optique (14), ces anses étant incurvées dans la même direction, selon le sens de rotation des aiguilles d'une montre. La partie terminale de chaque anse comporte une zone d'appui (25) arrondie. Grâce à cette disposition des anses, il est possible de disposer l'implant par rotation lors du placement dans l'oeil.

## Revendications

1/ Implant intra-oculaire (13) pour chambre antérieure de l'oeil, composé d'une partie optique (14) comprenant une lentille divergente à bords épais (16), de forme essentiellement circulaire, occupant substantiellement en totalité ladite partie optique, et présentant une face antérieure (17) et une face postérieure (18), et d'une partie haptique élastique permettant un appui à l'intérieur de l'oeil, et s'étendant à distance de la partie optique, **caractérisé en ce que**, la partie optique (14) étant construite de manière monobloc avec la partie haptique, en un matériau transparent, d'une part la partie haptique comprend deux anses (15) d'appui à l'intérieur de l'oeil, s'étendant de part et d'autre de la partie optique, selon la même direction diamétrale (50), à partir de deux points de la partie optique respectivement et diamétralement opposés, et d'autre part une zone de raccordement (19) de section décroissante vers l'extrémité libre de chaque anse, située à l'extérieur de la partie optique, et reportée sur chaque anse (15), relie cette dernière à la partie optique, ladite zone de raccordement présentant en tout point de sa surface extérieure un profil courbe.

2/ Implant selon la revendication 1, caractérisé en ce que chaque zone de raccordement (19) présente un bossage (20) disposé du côté de la face antérieure (17) de la lentille, au niveau du point de départ de chaque anse (15) d'appui élastique.

3/ Implant selon la revendication 1, caractérisé en ce que le bord épais (16) de la partie optique présente une épaisseur de deux à dix fois supérieure à celle des anses (15) d'appui élastique.

4/ Implant selon la revendication 1, caractérisé en ce que la zone de raccordement (19) présente une face postérieure tangente à la face postérieure (18) de la lentille (14).

5/ Implant selon la revendication 1, caractérisé en ce que chaque anse (15) d'appui élastique comporte, et de manière continue, successivement de son point de départ sur la partie optique (14), vers son extrémité libre, une zone de raccordement (19), une première partie courbe (23), sensiblement orthogonale à la direction diamétrale (50) d'extension de chaque dite anse, et une deuxième partie (24) s'étendant en direction opposée à ladite première partie courbe.

6/ Implant selon la revendication 5, caractérisé en ce que les deuxièmes parties (24) des deux anses (15) d'appui élastique respectivement sont disposées dans un plan postérieur sensiblement parallèle à la lentille (14), du côté de sa face postérieure (18).

7/ Implant selon la revendication 6, caractérisé en ce que les premières parties (23) des deux anses (15) d'appui élastique respectivement sont disposées dans un plan faisant un angle aigu α avec le plan postérieur des deuxièmes parties (24).

8/ Implant selon la revendication 7, caractérisé en ce que l'angle α est de l'ordre de 25°.

9/ Implant selon la revendication 1, caractérisé en ce que la longueur de la zone de raccordement (19) n'excède pas 10 % de la longueur totale de chaque anse (15) d'appui élastique.

10/ Implant selon la revendication 1, caractérisé en ce que la lentille de la partie optique présente une puissance comprise entre - 10 et - 70 dioptries.

**Patentansprüche**

1. Intraokulares Implantat (13) für die vordere Augenkammer, bestehend aus einem optischen Abschnitt (14), der eine im wesentlichen kreisförmige Zerstreuungslinse mit dickem Rand (16) aufweist, die im wesentlichen den optischen Abschnitt (14) einnimmt, wobei diese eine vordere Seite (17) und eine hintere Seite (18) aufweist, und ferner bestehend aus einem elastischen haptischen Abschnitt, der ein Abstützen im Augeninnern ermöglicht und der sich fern vom optischen Abschnitt erstreckt, dadurch gekennzeichnet, daß der optische Abschnitt (14) einstückig mit dem haptischen Abschnitt aus transparentem Material aufgebaut ist, daß einerseits der haptische Abschnitt im Augeninnern zwei Stützbügel (15) aufweist, die sich beidseits des optischen Abschnitts in derselben diametralen Richtung (50) erstrecken, und zwar ausgehend von zwei verschiedenen, diametral gegenüberliegenden Stellen des optischen Abschnittes, und daß andererseits ein, an der Außenseite des optischen Abschnitts angeordneter und über jeden Bügel (15) zurücktragender Übergangsbereich (19) mit in Richtung freies Ende jedes Bügels abnehmendem Querschnitt einen Bügel mit dem optischen Abschnitt verbindet, wobei der Übergangsbereich an jeder Stelle seiner Außenfläche ein gekrümmtes Profil aufweist.

2. Implantat nach Anspruch 1, dadurch gekennzeichnet, daß jeder Übergangsbereich (19) auf Höhe der Ausgangsstelle jedes elastischen Stützbügels (15) einen Buckel (20) aufweist, der auf der vorderen Seite (17) der Linse angeordnet ist.

3. Implantat nach Anspruch 1, dadurch gekennzeichnet, daß der dicke Rand (16) des optischen Abschnittes eine Dicke aufweist, die das Zwei- bis Zehnfache der Dicke der elastischen Stützbügel (15) beträgt.

4. Implantat nach Anspruch 1, dadurch gekennzeichnet, daß der Übergangsbereich (19) eine hintere Seite aufweist, die tangential zur hinteren Seite (18) der Linse (14) verläuft.

5. Implantat nach Anspruch 1, dadurch gekennzeichnet, daß jeder elastische Stützbügel (15) von der Ausgangsstelle vom optischen Abschnitt (14) in Richtung dessen freien Endes nacheinanderfolgend und durchgehend einen Übergangsbereich (19), einen ersten gekrümmten Bereich (23), der etwa orthogonal zur diametralen Ausdehnungsrichtung (50) jedes Bügels verläuft und einen zweiten Abschnitt (24) aufweist, der sich in entgegengesetzter Richtung zum ersten gekrümmten Abschnitt erstreckt.

6. Implantat nach Anspruch 5, dadurch gekennzeichnet, daß die zweiten Abschnitte (24) der beiden elastischen Stützbügel (15) in einer zurückversetzten Ebene angeordnet sind, die auf der hinteren Seite (18) parallel zur Linse (14) verläuft.

7. Implantat nach Anspruch 6, dadurch gekennzeichnet, daß die ersten Abschnitte (23) der beiden elastischen Stützbügel (15) jeweils in einer Ebene zum Liegen kommen, die unter einem spitzen Winkel α zur zurückversetzten Ebene der zweiten Abschnitte (24) steht.

8. Implantat nach Anspruch 7, dadurch gekennzeichnet, daß der Winkel α im Bereich von 25° liegt.

9. Implantat nach Anspruch 1, dadurch gekennzeichnet, daß die Länge des Übergangsbereichs (19) zehn Prozent der Gesamtlänge jedes elastischen Stützbügels (15) nicht überschreitet.

10. Implantat nach Anspruch 1, dadurch gekennzeichnet, daß die Linse des optischen Abschnittes eine Stärke zwischen - 10 und - 70 Dioptrien aufweist.

**Claims**

1. Intraocular implant (13) for the anterior chamber of the eye, consisting of an optic part (14) comprising a divengent lens with thick edges (16), of essentially circular shape, substantially completely occupying the said optic part, and having an anterior face (17) and a posterior face (18), and an elastic haptic part permitting a support inside the eye and extending away from the optic part, characterised in that, the optic part (14) being constructed in a monobloc manner with the haptic part and made of a transparent material, on the one hand the haptic part comprises two loops (15) for support inside the eye, extending on either side of the optic part, in the same diametral direction (50), from two points of the optic part respectively and diametrically opposed, and, on the other hand, a connecting zone (19) of cross-section decreasing towards the free end of each loop, situated outside the optic part, and fitted on each loop (15), connects the latter to the optic part, the said connecting zone having a curved profile over its entire outer surface.

2. Implant according to Claim 1, characterized in that each connecting zone (19) has a hump (20) arranged next to the anterior face (17) of the lens, at the point of departure of each loop (15) for elastic support.

3. Implant according to Claim 1, characterized in that the thick edge (16) of the optic part has a thickness twice to ten times greater than that of the loops (15) for elastic support.

4. Implant according to Claim 1, characterized in that the connecting zone (19) has a posterior face tangential to the posterior face (18) of the lens (14).

5. Implant according to Claim 1, characterized in that each loop (15) for elastic support comprises, continuously and successively from its point of departure on the optic part (14), towards its free end, a connecting zone (19), a first curved part (23), substantially orthogonal to the diametral direction (50) of extension of each said loop, and a second part (24) extending in the opposite direction to the said first curved part.

6. Implant according to Claim 5, characterized in that the second parts (24) of the two loops (15) for elastic support are each arranged in a posterior plane substantially parallel to the lens (14), next to its posterior face (18).

7. Implant according to Claim 6, characterized in that the first parts (23) of the two loops (15) for elastic support are each arranged in a plane forming an acute angle α to the posterior plane of the second parts (24).

8. Implant according to Claim 7, characterized in that the angle α is of the order of 25°.

9. Implant according to Claim 1, characterized in that the length of the connecting zone (19) does not exceed 10% of the total length of each loop (15) for elastic support.

10. Implant according to Claim 1, characterised in that the lens of the optic part has a power of between -10 and -70 dioptres.

FIG_1

FIG_2

FIG_3

FIG_4

FIG_5

# FIG.6